(19) Europäisches Patentamt
European Patent Office
Office européen des brevets

(11) Veröffentlichungsnummer : **0 347 690 B1**

# (12) EUROPÄISCHE PATENTSCHRIFT

(45) Veröffentlichungstag der Patentschrift :
**21.10.92 Patentblatt 92/43**

(51) Int. Cl.[5] : **C25B 3/02,** C07C 33/46, C07C 69/025

(21) Anmeldenummer : **89110558.7**

(22) Anmeldetag : **10.06.89**

## (54) Verfahren zur Herstellung von Benzolderivaten und neue Benzolderivate.

(30) Priorität : **21.06.88 DE 3820897**

(43) Veröffentlichungstag der Anmeldung :
**27.12.89 Patentblatt 89/52**

(45) Bekanntmachung des Hinweises auf die Patenterteilung :
**21.10.92 Patentblatt 92/43**

(84) Benannte Vertragsstaaten :
**AT BE CH DE FR GB IT LI NL**

(56) Entgegenhaltungen :
**US-A- 3 448 021**
**US-A- 3 988 339**
**US-A- 4 402 804**

(73) Patentinhaber : **BASF Aktiengesellschaft**
**Carl-Bosch-Strasse 38**
**W-6700 Ludwigshafen (DE)**

(72) Erfinder : **Hermeling, Dieter, Dr.**
**Zum Ordenswald 73 f**
**W-6730 Neustadt (DE)**
Erfinder : **Degner, Dieter, Dr.**
**Kurpfalzstrasse 8**
**W-6701 Dannstadt-Schauernheim (DE)**
Erfinder : **Harreus, Albrecht, Dr.**
**Teichgasse 13**
**W-6700 Ludwigshafen (DE)**
Erfinder : **Goetz, Norbert, Dr.**
**Schoefferstrasse 25**
**W-6520 Worms 1 (DE)**
Erfinder : **Wild, Jochen, Dr.**
**An der Marlach 7**
**W-6705 Deidesheim (DE)**
Erfinder : **Theobald, Hans, Dr.**
**Queichstrasse 6**
**W-6703 Limburgerhof (DE)**
Erfinder : **Wolf, Bernd, Dr.**
**Eichenstrasse 11**
**W-6704 Mutterstadt (DE)**

**EP 0 347 690 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Diese Erfindung betrifft ein neues elektrochemisches Verfahren zur Herstellung von Benzolderivaten sowie neue Benzolderivate. Nach dem neuen Verfahren erhält man aus Toluolderivaten Benzylester, die z.B. für die Herstellung von Benzylalkoholen verwendet werden können. Die neuen Benzolderivate dieser Erfindung sind Benzylester und Benzylalkohole.

Es wurde gefunden, daß man Benzylester der allgemeinen Formel

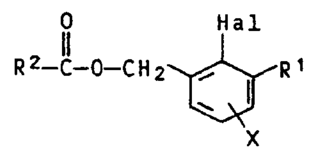

I,

in der Hal ein Halogenatom, $R^1$ einen geradkettigen, verzweigten oder ringförmigen Kohlenwasserstoffrest mit 1 bis 18 C-Atomen, $R^2$ ein Wasserstoffatom oder einen Alkylrest mit 1 bis 6 C-Atomen und X ein Wasserstoff- oder Halogenatom bedeuten, vorteilhaft dadurch herstellen kann, daß man Toluolderivate der allgemeinen Formel

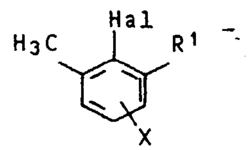

II,

in der Hal, $R^1$ und X die obengenannte Bedeutung haben, in Gegenwart einer Säure der Formel $R^2$-COOH (III) elektrochemisch oxidiert.

Bekanntlich kann man Benzylalkohole und deren Ester mit niederen Alkancarbonsäuren nach den in Houben-Weyl, Methoden der Organischen Chemie, beschriebenen Verfahren herstellen, z.B. durch

a) Bromierung von Methylaromaten mit N-Bromsuccinimid (s. Band 4, 221) und nachfolgende Reaktion des Bromierungsproduktes mit Salzen von Carbonsäuren und anschließende Verseifung,

b) Acetoxylierung von Methylaromaten mit Blei-(IV)-oder Cer-(IV)-Verbindungen oder durch Oxidation mit molekularem Sauerstoff,

c) Oxidation von Methylaromaten zu den entsprechenden Benzaldehyden bzw. Benzoesäuren (E3 bzw. IV/1b) und anschließende Reduktion zu den Alkoholen, oder

d) Bildung einer metallorganischen Verbindung aus einem halogenierten Aromaten und nachfolgende Umsetzung mit Formaldehyd.

Für die Herstellung der Verbindungen der Formel I sind diese bekannten Methoden entweder nicht oder wegen ihrer mangelhaften Selektivität wenig geeignet. So werden die Ausgangsstoffe der Formel II durch die genannten Oxidationsmittel gemäß (a) bis (c) sowohl an der Methylgruppe als auch am aliphatischen Rest $R^1$ angegriffen. Ausgangsstoffe mit zwei Halogenatomen unterliegen bei der Bildung der metallorganischen Zwischenstufe gemäß (d) ebenfalls einer mangelhaften Differenzierung. Man erhält somit Reaktionsgemische, aus denen die gewünschten Verbindungen der Formel I nur schwer isoliert werden können.

Nach dem elektrochemischen Verfahren der Erfindung erhält man die Benzylester demgegenüber mit hoher Selektivität.

Die für das erfindungsgemäße Verfahren benötigten Toluolderivate der Formel II enthalten als Rest $R^1$ einen geradkettigen, verzweigten oder ringförmigen Kohlenwasserstoffrest mit 1 bis 18 C-Atomen. Kohlenwasserstoffreste dieser Art sind insbesondere geradkettige und verzweigte Alkylreste sowie Cycloalkylreste. Als geradkettige Alkylreste seien z.B. Methyl, Ethyl, Propyl, Butyl, Pentyl, Hexyl ... genannt. Verzweigte Alkylreste sind z.B. solche der Formel

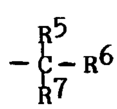

die insgesamt mindestens 3 C-Atome enthalten und wobei die Reste $R^5$, $R^6$ und $R^7$ für Wasserstoffatome oder Alkylreste mit 1 bis 6, vorzugsweise 1 bis 3 C-Atomen, stehen können. Als Reste dieser Art seien z.B. Isopropyl,

tert.-Butyl, sek.-Butyl und iso-Butyl genannt. Cycloalkylreste sind solche, die eine Cycloalkylgruppe oder eine Bicycloalkylgruppe aufweisen. Die Cycloalkylgruppen enthalten z.B. 3 bis 8 cyclische C-Atome, die ihrerseits Alkylgruppen mit 1 bis 5, vorzugsweise 1 bis 3 C-Atomen tragen können. Cycloalkylreste dieser Art sind z.B. Cyclopropyl, Cyclopentyl, Cyclohexyl, 3,5-Diethylcyclohexyl oder Tetramethylcyclopropyl. Die Bicycloalkylgruppen enthalten z.B. 5 bis 12 bicyclische C-Atome, die ihrerseits Alkylgruppen mit 1 bis 5, vorzugsweise 1 bis 3 C-Atomen enthalten können. Als Bicycloalkylgruppen sind z.B. im einzelnen genannt: 2-Norbornyl, Bicyclo[4.1.0]hept-1-yl oder 2,6-Dimethyl-bicyclo[4.1.0]hept-1-yl.

Die in den Toluolderivaten der Formel II enthaltenen Halogenatome sind bevorzugt Fluor-, Chlor- oder Bromatome. Als Toluolderivate der Formel II sind z.B. die folgenden Verbindungen zu nennen:

2-Chlor-3-isopropyl-toluol, 2-Fluor-3-isopropyl-toluol,
2-Brom-3-isopropyl-toluol, 2-Chlor-3-cyclohexyl-toluol,
3-Cyclopentyl-2-fluor-toluol, 2-Chlor-3-cyclopentyl-toluol,
2-Chlor-3-tert.-butyl-toluol, 2-Fluor-3-tert.-butyl-toluol,
2-Chlor-3-sec.-butyl-toluol, 2-Chlor-6-fluor-3-isopropyl-toluol.

Man kann die Toluole z.B dadurch herstellen, daß man die Aminogruppe von in 2,6-Stellung dialkylierten Anilinen durch Diazotierung und Sandmeyer- oder Balz-Schiemann-Reaktion gegen das Halogenatom austauscht. Die in 2,6-Stellung dialkylierten Aniline kann man entweder aus den analogen Phenolen (EP 53 696) oder durch selektive ortho-Alkylierung von Anilinen erhalten (US-PS 3,678,12, DE-OS 3 309 354).

Eine weitere Syntehesemöglichkeit für die Aniline ist durch die Amino-Claisen-Umlagerung gegeben (Izv. Akad. Nauk.SSSR, Ser. Khim. 1982, 2160 ff). Die dabei anfallenden ungesättigten Aniline werden dann selektiv in der Seitenkette hydriert.

Die Toluolderivate der Formel II werden in Gegenwart von Alkansäuren der Formel III, von denen Ameisen-, Essig- und Propionsäuren bevorzugt sind, elektrochemisch zu deren Verbindungen der Formel I oxidiert.

Die elektrochemische Oxydation von einfach substituierten Toluolen wird z.B. in der US-PS 3 448 021; Acta Chem. Scand. B 32, 157 (1978); Acta Chem. Scand. B 33, 113 (1979) und J. Org. Chem. 51, 4544 (1986) beschrieben. Demgegenüber ist es überraschend, daß bei dem erfindungsgemäßen Verfahren die Toluole der Formel II, die außer der Methylgruppe noch weitere Alkylreste enthalten, unter selektiver Oxidation der Methylgruppen glatt in die Verbindungen der Formel I übergehen.

Die elektrochemische Oxidation kann in den üblichen Elektrolysezellen durchgeführt werden, bevorzugt werden ungeteilte Durchflußzellen eingesetzt. Als Anodenmaterialien dienen z.B. Edelmetalle, wie Platin oder Oxide wie $RuO_2$, $Cr_2O_3$ oder $TiO_x/RuO_x$; besonders bevorzugt wird Graphit eingesetzt. Kathodenmaterialien können u. a. Eisen, Stahl, Nickel, Edelmetalle, wie Platin oder auch Graphit sein. Als Elektrolyt dient eine Lösung der Toluole in der Alkansäure, dem zur Erhöhung der Leitfähigkeit ein Hilfselektrolyt zugesetzt wird. Als Hilfselektrolyte können die in der Elektrochemie üblichen Leitsalze, wie z.B. Fluoride, Tetrafluoroborate, Sulfonate oder Alkylsulfate eingesetzt werden. Weiterhin kann auch zur Erhöhung der Löslichkeit der Toluole ein Colösungsmittel zugesetzt werden. Beispiele für Colösungsmittel sind Ketone, wie Aceton, Methylethylketon, Nitrile, wie Acetonitril oder Propionitril und Anhydride, wie Acetanhydrid.

Der Elektrolyt hat beispielsweise folgende Zusammensetzung:

1 - 20 % Toluol der allgemeinen Formel II
1 - 10 % Leitsalz
0 - 20 % Colösungsmittel
50 - 95 % Alkansäure

Die Stromdichten und Elektrolysetemperaturen lassen sich in weiten Grenzen variieren. So wird z.B. bei 0.2 - 20 $A/dm^2$ und bei 15 bis 95°C elektrolysiert. Die eingesetzten Toluole lassen sich weitgehend umsetzen, die Aufarbeitung der Elektrolyseausträge erfolgt nach an sich bekannten Methoden, z.B. durch Destillation, Extraktion und Kristallisation. Colösungsmittel, überschüssige Alkansäure und Leitsalz können nach Abtrennung von den Benzylestern zusammen mit ggf. unumgesetztem Toluol der Formel II zur Elektrolyse zurückgeführt werden.

Die Erfindung betrifft außerdem die neuen Benzolderivate der allgemeinen Formel

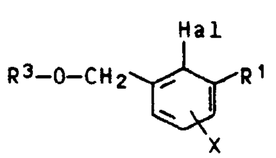 IV,

in der Hal ein Halogenatom, $R^1$ einen geradkettigen, verzweigten oder ringförmigen Kohlenwasserstoffrest mit 1 bis 18 C-Atomen, $R^3$ ein Wasserstoffatom oder einen $R^2$-CO-Rest, $R^2$ ein Wasserstoffatom oder einen Alkylrest mit 1 bis 6 C-Atomen und X ein Wasserstoff- oder Halogenatom bedeuten, wobei jedoch $R^1$ nicht Methyl sein kann, wenn X Wasserstoff bedeutet.

Verbindungen dieser Art sind z.B. die in der folgenden Tabelle aufgeführten Benzolderivate der Formel IV

| Nr. | Hal | $R^1$ | $R^3$ | X |
|---|---|---|---|---|
| 1 | Cl | 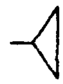 | $CH_3-CO-$ | H |
| 2 | Cl | $-CH(CH_3)-CH_2-CH_3$ | $CH_3-CO-$ | H |
| 3 | Br | $-CH(CH_3)_2$ | H | H |
| 4 | Br | $-CH(CH_3)_2$ | $CH_3-CO-$ | H |
| 5 | Cl | 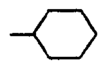 | $CH_3-CO-$ | H |
| 6 | Cl | 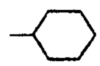 | H | H |
| 7 | F | $-CH(CH_3)_2$ | H | H |
| 8 | F | $-CH(CH_3)_2$ | $CH_3-CO-$ | H |
| 9 | Cl | 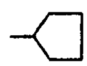 | H | H |
| 10 | Cl | 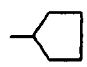 | $CH_3-CO-$ | H |
| 11 | F | 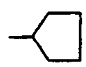 | H | 6-Cl |
| 12 | F | 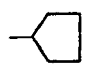 | $CH_3-CO-$ | 6-Cl |
| 13 | Cl | $-CH(CH_3)_2$ | $CH_3-CO-$ | H |
| 14 | Cl | $-CH(CH_3)_2$ | H | H |
| 15 | Cl | $-CH(CH_3)_2$ | $CH_3-CO-$ | 6-F |
| 16 | Cl | $-CH(CH_3)_2$ | H | 6-F |
| 17 | Cl | $-C(CH_3)_3$ | $CH_3-CO-$ | H |
| 18 | Cl | $-C(CH_3)_3$ | H | H |
| 19 | Cl | $-CH(CH_3)-CH_2-CH_3$ | H | H |
| 20 | F | 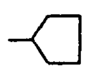 | $CH_3-CO-$ | H |
| 21 | F | 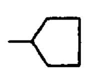 | H | H |
| 22 | Cl | $-CH(CH_3)_2$ | $H-CO-$ | H |
| 23 | Cl | $-CH(CH_3)_2$ | $CH_3-CH_2-CO-$ | H |
| 24 | F | $-C(CH_3)_3$ | $CH_3-CO-$ | H |
| 25 | F | $-C(CH_3)_3$ | H | H |

Von besonderem technischen Interesse sind die neuen Benzolderivate, der allgemeinen Formel

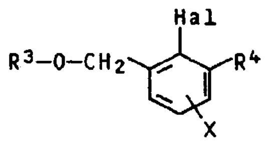

V,

in der Hal ein Fluor-, Chlor- oder Bromatom und $R^4$ einen verzweigten oder ringförmigen Alkylrest mit 3 bis 12, vorzugsweise 3 bis 8 C-Atomen bedeuten und $R^3$ und X die oben genannte Bedeutung haben.

Die Verbindungen der allgemeinen Formeln IV und V sind wichtige Zwischenprodukte für die Synthese von Pflanzenschutzmitteln, insbesondere von neuen Pyrethroiden wie sie in der EP-A-347 694 beschrieben werden. Dabei geht man im allgemeinen von den Benzylalkoholen der Formel

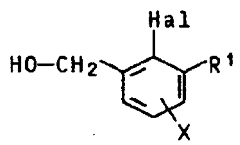

VII

aus, in der Hal, $R^1$ und X die obengenannte Bedeutung haben. Diese Benzylalkohole gewinnt man aus den Benzylestern der Formel IV, in der $R^3$ für den Rest $R^2$-CO- steht, durch Hydrolyse. Man hydrolysiert z.B. nach an sich bekannten Verfahren. Der betreffende Benzylester wird dazu in einem inerten organischen Lösungsmittel, wie Tetrahydrofuran, Dioxan, Ethanol, Methanol mit einer Base, wie Natriumhydroxid hydrolysiert, gegebenenfalls unter Zusatz von Wasser. Die saure Hydrolyse eines Benzylesters erfolgt beispielsweise durch Umsetzung in einem der obigen Lösungsmittel unter Zusatz von Wasser und einer Säure, beispielsweise Salzsäure.

Die Hydrolysen werden bevorzugt bei Raumtemperatur bis zum Siedepunkt des Reaktionsgemisches durchgeführt.

Beispiel 1

2-Chlor-3-isopropyl-benzylalkohol

a) Herstellung von 2-Chlor-3-isopropyl-toluol

224 g (1,5 mol) 2-Isopropyl-6-methyl-anilin werden in 900 ml halbkonzentrierte Salzsäure gegeben. Bei einer Innentemperatur von 0 bis 5°C wird eine Lösung von 113,9 g (1,65 mol) Natriumnitrit in 500 ml Wasser zugetropft. Die so hergestellte Reaktionsmischung gibt man bei 0 bis 5°C portionsweise zu einer Mischung aus 298 g (2 mol) frisch hergestelltem Kupfer(I)chlorid und 800 ml konzentrierter Salzsäure. Die Temperatur des Reaktionsgemisches läßt man auf Raumtemperatur ansteigen und erwärmt zur Vervollständigung der Umsetzung noch auf 90°C, bis die Stickstoffentwicklung beendet ist. Die Reaktionsmischung wird dann einer Wasserdampfdestillation unterworfen. Man erhält durch Extraktion mit Dichlormethan 199 g eines Rohproduktes, das nach GC-Analyse zu 48,3 % aus 2-Chlor-3-isopropyl-toluol und zu 42,2 % aus 2-Isopropyl-6-methyl-phenol besteht. Man löst 30 g (0,75 mol) Natriummethylat in 250 ml Methanol und tropft das Rohprodukt zu, rührt 3 h bei 50°C, engt ein, versetzt zweimal mit Toluol und engt jeweils ein. Der Rückstand wird mit Cyclohexan suspendiert und über Kieselgel abfiltriert. Nach Einengen des Filtrates und Destillation erhält man: 87 g (34 %) 2-Chlor-3-isopropyl-toluol (Kp = -115 - 120°C (15 mbar))

b) Elektrosynthese von 2-Chlor-3-isopropyl-benzylacetat

Man elektrolysiert das nach Absatz a) erhaltene 2-Chlor-3-isopropyl-toluol in der wie folgt beschriebenen Elektrolysezelle unter den angegebenen Bedingungen.

Apparatur: ungeteilte Zelle mit 11 bipolaren Elektroden

Anode: Graphit

Elektrolyt: 186 g (1,104 Mol) 2-Chlor-3-isopropyl-toluol, 300 g Acetanhydrid, 90 g $KSO_3C_6H_5$ und 2424 g Essigsäure

Kathode: Graphit

Stromdichte: 0,67 A/dm$^2$

Elektrolysetemperatur: 70°C

Elektrolyse mit 7 F/Mol 2-Chlor-3-isopropyl-toluol. Der Elektrolyt wird während der Elektrolyse mit 200 l/h durch die Zelle gepumpt.

Nach Beendigung der Elektrolyse werden Essigsäure/Acetanhydrid bei Normaldruck bis 150°C abdestilliert und der Rückstand zwischen $H_2O$/Methyl-tert.-butylether verteilt. Aus der organischen Phase wird Methyl-tert.-butylether bei Normaldruck abdestilliert. Man erhält 355 g Rückstand, der nach gaschromatographischer Analyse 11,7 g 2-Chlor-3-isopropyl-toluol und 165,7 g 2-Chlor-3-isopropyl-benzylacetat enthält. Hieraus errechnet sich ein Umsatz von 93,7 %, eine Ausbeute von 66,3 %, sowie eine Selektivität von 70,7 %. 2-Chlor-3-isopropyl-benzylacetat wird bei 2 mbar und 92-94°C reindestilliert.

$^1$H-NMR (270 MHz, $CDCl_3$)

δ (ppm) = 1,25 (d, 6H, -CH(C$\underline{H}_3$)$_2$); 2,14 (s, 3H, $CH_3COO$-); 3,48 (Septett, 1H, -C$\underline{H}$($CH_3$)$_2$); 5,23 (s, 2H, -$CH_2$OAc); 7,28 (m, 3H, aromat.-H).

c) Herstellung von 2-Chlor-3-isopropyl-benzylalkohol

70 g des nach Absatz b) gewonnenen Benzylesters werden in 500 ml Dioxan gelöst. Die Lösung wird mit 180 g 8 %iger Natronlauge versetzt. Man rührt 5 h bei 50°C, engt im Vakuum ein, nimmt mit Wasser auf und extrahiert das wäßrige Gemisch mehrfach mit Dichlormethan. Aus der organischen Phase gewinnt man nach Trocknen und Einengen: 57,0 g (quant.) 2-Chlor-3-isopropyl-benzylalkohol. (Kp = 87-89°C, 18 mbar).

$^1$H-NMR (250 MHz, $CDCl_3$)

δ (ppm) = 1,25 (d, 6H, -CH(C$\underline{H}_3$)$_2$); 2,1 (m, 1H, OH); 3,45 (m, 1H, -CH<); 4,8 (d, 2H, -$CH_2$); 7,2-7,4 (m, 3H, aromat.-H).

Beispiel 2

2-Chlor-3-cyclopentyl-benzylalkohol

a) Herstellung von 2-Cyclopentyl-6-methyl-phenol

Zu 1,08 kg (10 mol) o-Kresol in 400 ml Pentan werden 170 ml Bortrifluoridphosphorsäureaddukt gegeben. Bei Rückflußtemperatur werden innerhalb 3 h 680 g (10 mol) Cyclopenten, gelöst in 1 l Pentan, zugetropft. Die Mischung wird noch 1 h bei Rückflußtemperatur gerührt und anschließend in Eiswasser gegossen. Die organische Phase wird abgetrennt und 4mal mit je 600 ml 10 %ier Natronlauge extrahiert. Die organische Phase wird getrocknet und eingeengt, der Rückstand wird destilliert (84-88°C/0,2 mbar). Man erhält 515 g 2-Cyclopentyl-6-methyl-phenol (entspricht 37 % der Theorie, bezogen auf umgesetztes Kresol). Aus der alkalischen Waschlösung erhält man nach Ansäuern, Extraktion und Destillation: 217 g o-Kresol und 282 g 4-Cyclopentyl-2-methyl-phenol.

b) Herstellung von 2-Cyclopentyl-6-methyl-cyclohexylamin

In einem 5-l-Rührautoklaven wird eine Mischung aus 485 g 2-Cyclopentyl-6-methyl-phenol und 551 g Ammoniak in Gegenwart eines Pulverkatalysators der Zusammensetzung 10 Gew.% Palladium, 5 Gew.% Praseodymoxid auf Aluminiumoxid bei einer Temperatur von 230°C und einem Wasserstoffdruck von 300 bar bis zur Druckkonstanz reduktiv aminiert. Nach Abtrennung des Katalysators werden 484 g rohes 2-Cyclopentyl-6-methyl-cyclohexylamin (95 % Ausbeute) erhalten, die ohne weitere Reinigung in die nächste Stufe eingesetzt werden können.

c) Herstellung von 2-Cyclopentyl-6-methyl-anilin

In ein druckfestes zylindrisches Rohr mit einem Volumen von 0,25 l als Reaktor wird ein Strangkatalysator (3 mm Durchmesser, 10 mm Länge), der 0,5 Gew.% Palladium auf einer Mischung aus 19,4 Gew.% Magnesiumoxid und 80,6 Gew.% Aluminiumoxid enthält, eingefüllt. Man erhitzt auf 220°C. Dann werden stündlich bei Atmosphärendruck 60 g 2-Cyclopentyl-6-methyl-cyclohexylamin und gleichzeitig eine Gasmischung aus 200 Nl Ammoniak und 300 Nl Wasserstoff im Gleichstrom durch den Rohrreaktor geleitet. Das Reaktionsprodukt wird, sobald es den Reaktor verläßt, abgekühlt. Es besteht nach gaschromatographischer Analyse aus 44 Gew.% 2-Cyclopentyl-6-methyl-cyclohexylamin (Kp = 132°C, 18 mbar) und 54 Gew.% 2-Cyclopentyl-6-methyl-anilin (Kp = 160°C, 18 mbar). Diese beiden Amine können durch Destillation leicht voneinander getrennt werden.

d) Herstellung von 2-Chlor-3-cyclopentyl-toluol

173 g (0,96 mol) 2-Cyclopentyl-6-methyl-anilin werden entsprechend Beispiel 1, Absatz a) diazotiert und mit Kupfer(I)chlorid umgesetzt. Nach Wasserdampfdestillation erhält man ein Rohprodukt von 100,1 g, das nach GC-Analyse zu 56,4 % aus 2-Chlor-3-cyclopentyl-toluol und 32 % aus 2-Chlor-3-cyclopentyl-phenol besteht. Das Phenol wird entsprechend Beispiel 1, Absatz a) abgetrennt und kann erneut entsprechend Absatz b) umgesetzt werden. Man erhält: 60,4 g 2-Chlor-3-cyclopentyl-toluol vom Kp = 140°C (0,5 mbar).

e) Elektrosynthese von 2-Chlor-3-cyclopentyl-benzylacetat

Man elektrolysiert das nach Absatz d) erhaltene 2-Chlor-3-cyclopentyl-toluol in der wie folgt beschriebenen Elektrolysezelle unter den angegebenen Bedingungen.

Apparatur: ungeteilte Zelle mit 11 bipolaren Elektroden

Anode: Graphit

Elektrolyt: 41 g (0,211 Mol) 2-Chlor-3-cyclopentyl-toluol, 350 g Acetanhydrid, 175 g $KSO_3C_6H_5$ und 2934 g Essigsäure

Kathode: Graphit

Stromdichte: 1,33 $A/dm^2$

Elektrolysetemperatur: 70-75°C

Elektrolyse mit 9 F/Mol 2-Chlor-3-cyclopentyl-toluol.

Der Elektrolyt wird während der Elektrolyse mit 200 l/h durch die Zelle gepumpt.

Der Elektrolyseaustrag wird analog Beispiel 1, Absatz b) aufgearbeitet. Der Rückstand (105 g) enthält nach GC-Analyse, 7,0 g 2-Chlor-3-cyclopentyl-toluol und 37,1 g 2-Chlor-3-cyclopentyl-benzylacetat. Hieraus errechnet sich ein Umsatz von 82,8 %, eine Ausbeute von 69,6 % und eine Selektivität von 84,1 %. 2-Chlor-3-cyclopentyl-benzylacetat wird bei 4 mbar und 140-150°C reindestilliert.

$^1H$-NMR (270 MHz, $CDCl_3$)

$\delta$ (ppm) = 1,45-1,9 (m, 8H, Cyclopentyl-$CH_2$); 2,1 (s, 3H, $CH_3COO$-); 3,5 (Quintett, 1H, Cyclopentyl-CH); 5,2 (s, 2H, -$CH_2OAc$); 7,2 (m, 3H, aromat.-H).

$^{13}C$-NMR (270 MHz, $CDCl_3$)

$\delta$ (ppm) = 20,7 (q), 25,5 (t, 2C); 33,3 (t, 2C); 42,3 (d); 64,3 (t); 126,6 (d); 126,9 (d); 127,0 (d); 133,5 (s); 134,1 (s); 144,4 (s); 170,4 (s).

f) Herstellung von 2-Chlor-3-cyclopentyl-benzylalkohol

8,1 g 2-Chlor-3-cyclopentyl-benzylacetat werden analog Beispiel 1, Absatz c) verseift. Man erhält 2-Chlor-3-cyclopentyl-benzylalkohol vom Kp: 123 bis 125°C (0,2 mbar) in einer Ausbeute von 5,8 g (86 %).

$^1H$-NMR (200 MHz, $CDCl_3$)

$\delta$ (ppm) = 1,4-2,3 (m, 9H, Cyclopentyl-$CH_2$ und -OH); 3,5 (m, 1H, -CH); 4,8 (s, 2H, $CH_2$-OH); 7,2-7,4 (m, 3H, aromat.-H).

Beispiel 3

2-Fluor-3-isopropyl-benzylalkohol

a) Herstellung von 2-Fluor-3-isopropyl-toluol

In 200 ml 40 %ige Tetrafluoroborsäure tropft man bei 5°C 44,7 g (0,3 mol) 2-Methyl-6-isopropyl-anilin und anschließend eine Lösung von 20,7 g Natriumnitrit in 20 ml Wasser. Die Mischung wird 1 h bei 10°C gerührt. Das ausgefallene Diazoniumtetrafluoroborat wird abgesaugt, nacheinander mit 50 ml Eiswasser, 50 ml kaltem Ethanol und 50 ml Diethylether suspendiert. Die Suspension wird in einen auf 150°C vorgeheizten Kolben getropft. Nach Beendigung der Gasentwicklung wird das Produkt durch Destillation über eine Claisendestille (Badtemperatur 100°C, Druck 4 mbar, Übergangstemperatur 41-42°C) gewonnen. Man erhält 28 g (61 %) 2-Fluor-3-isopropyl-toluol.

b) Elektrosynthese von 2-Fluor-3-isopropyl-benzylacetat

Man elektrolysiert das nach Absatz a) erhaltene 2-Fluor-3-isopropyl-toluol in der wie folgt beschriebenen Elektrolysezelle unter den angegebenen Bedingungen.

Apparatur: ungeteilte Zelle mit 11 bipolaren Elektroden
Anode: Graphit
Elektrolyt: 131 g (0,862 Mol) 2-Fluor-3-isopropyl-toluol, 350 g Acetanhydrid, 175 g $KSO_3C_6H_5$
Kathode: Graphit
Stromdichte: 1,33 A/$dm^2$
Elektrolysetemperatur: 70°C
Elektrolyse mit 5 F/Mol 2-Fluor-3-isopropyl-toluol.
Der Elektrolyt wird während der Elektrolyse mit 200 l/h durch die Zelle gepumpt.

Der Elektrolyseaustrag wird analog Beispiel 1, Absatz b) aufgearbeitet. Der Rückstand (324 g) enthält nach GC-Analyse 1,3 g 2-Fluor-3-isopropyl-toluol und 94,3 g 2-Fluor-3-isopropyl-benzylacetat. Hieraus errechnet sich ein Umsatz von 99 %, eine Ausbeute von 52,1 % sowie eine Selektivität von 52,6 %. 2-Fluor-3-isopropyl-benzylacetat wird bei 2 mbar und 74°C reindestilliert.

$^1$H-NMR (270 MHz, $CDCl_3$)

δ (ppm) = 1,25 (d, 6H, -CH(C$\underline{H}_3$)$_2$); 2,1 (s, 3H, $CH_3$COO-); 3,26 (Septett, 1H, -C$\underline{H}$($CH_3$)$_2$); 5,18 (s, 2H, -$CH_2$OAc); 7,05-7,3 (m, 3H, aromat.-H).

c) Herstellung von 2-Fluor-3-isopropyl-benzylalkohol

Zu einer Lösung von 15,3 g (73 mmol) 2-Fluor-3-isopropyl-benzylacetat in 100 ml Dioxan gibt man 44 g 8 %ige Natronlauge. Nach 5 h bei 50°C wird die Mischung in 100 ml Wasser gegossen. Durch Extraktion mit Dichlormethan erhält man 12 g (98 %) 2-Fluor-3-isopropyl-benzylalkohol.

$^1$H-NMR (200 MHz, $CDCl_3$)

δ (ppm) = 1,25 (d, 6H, -CH(C$\underline{H}_3$)$_2$); 2,13 (s, 1H, -OH); 3,26 (Septett, 1H, -C$\underline{H}$($CH_3$)$_2$); 4,75 (s, 2H, -$CH_2$OH); 7,05-7,29 (m, 3H, aromat.-H).

Beispiel 4

2-Brom-3-isopropyl-benzylalkohol

a) Herstellung von 2-Brom-3-isopropyl-toluol

Ausgehend von 2-Isopropyl-6-methyl-anilin erhält man analog Beispiel 1, Absatz a) durch Sandmeyer-Reaktion mit Kupfer(I)bromid 2-Brom-3-isopropyl-toluol vom Kp: 105-110°C (10 mbar).

b) Elektrosynthese von 2-Brom-3-isopropyl-benzylacetat

Man elektrolysiert das nach Absatz a) erhaltene 2-Brom-3-isopropyl-toluol in der wie folgt beschriebenen Elektrolysezelle unter den angegebenen Bedingungen.

Apparatur: ungeteilte Zelle mit 11 bipolaren Elektroden
Anode: Graphit
Elektrolyt: 207 g (0,972 Mol) 2-Brom-3-isopropyl-toluol, 350 g Acetanhydrid, 175 g $KSO_3C_6H_5$ und 2763 g Essigsäure
Kathode: Graphit
Stromdichte: 1,33 A/$dm^2$
Elektrolysetemperatur: 70-75°C
Elektrolyse mit 9 F/Mol 2-Brom-3-isopropyl-toluol.
Der Elektrolyt wird während der Elektrolyse mit 200 l/h durch die Zelle gepumpt.

Der Elektrolyseaustrag wird analog Beispiel 1, Absatz b) aufgearbeitet. Der Rückstand (309 g) enthält nach GC-Analyse 26,3 g 2-Brom-3-isopropyl-toluol und 153,9 g 2-Brom-3-isopropyl-benzylacetat. Hieraus errechnet sich ein Umsatz von 87,3 %, eine Ausbeute von 58,4 % und eine Selektivität von 66,9 %. 2-Brom-3-isopropyl-benzylacetat wird bei 4 mbar und 130°C reindestilliert.

$^1$H-NMR (270 MHz, $CDCl_3$)

δ (ppm) = 1,23 (d, 6H, -CH(C$\underline{H}_3$)$_2$); 2,1 (s, 3H, $CH_3$COO-); 3,47 (Septett, 1H, -C$\underline{H}$($CH_3$)$_2$); 5,2 (s, 2H, -$CH_2$OAc); 7.25 (m, 3H, aromat.-H).

$^{13}$C-NMR (270 MHz, $CDCl_3$)

δ (ppm) = 20,7 (q); 22,9 (q, 2C); 33,0 (d); 66,7 (t); 125,2 (s); 126,4 (d); 127,2 (d); 127,4 (d); 135,8 (s); 148,1 (s); 170,3 (s).

c) Herstellung von 2-Brom-3-isopropyl-benzylalkohol

116 g (0,43 mol) 2-Brom-3-isopropyl-benzylacetat werden in 770 ml Dioxan und 255 g 8 %iger Natronlauge analog Beispiel 1, Absatz c) hydrolysiert und aufgearbeitet. Man erhält 98,2 g (quant.) 2-Brom-3-isopropyl-benzylalkohol vom Kp. 110-112°C (0,2 mbar).

$^1$H-NMR (300 MHz, $CDCl_3$):

δ (ppm) = 1,25 (d, 6H, -CH(C$\underline{H}_3$)$_2$); 2,2 (s, 1H, OH); 3.48 (Septett, 1H, -CH<); 4,75 (s, 2H, $-CH_2-$); 7,2-7,4 (m, 3H, aromat.-H).

Beispiel 5

2-Chlor-3-cyclohexyl-benzylalkohol

a) Herstellung von 2-Cyclohexyl-6-methyl-anilin

Bei Raumtemperatur tropft man unter Rühren 63,1 g (0,52 mol) 3-Chlorcyclohexen zu 278,6 g (2,6 mol) o-Toluidin, erwärmt das Gemisch anschließend auf 160°C und hält es 6 h bei dieser Temperatur. Nach dem Abkühlen wird das Gemisch in Dichlormethan aufgenommen und mit Natronlauge gewaschen. Aus der organischen Phase gewinnt man nach Trocknen, Einengen und Rektifikation 76,2 g einer Mischung der beiden Aniline (92 % 2-(Cyclohexen-3-yl)-6-methyl-anilin und 4 % 4-(Cyclohexen-3-yl)-2-methyl-anilin).

223,3 g (1,15 mol) des Anilingemisches wird in 2 l Ethanol gelöst und mit 10 g Palladium/Kohle (10 %) bei Raumtemperatur und 0,2 bar Überdruck hydriert. Nach Abfiltrieren des Katalysators und Einengen erhält man 217 g Rohprodukt, das laut GC zu 92 % aus 2-Cyclohexyl-6-methyl-anilin besteht. Durch Überführung ins Hydrochlorid und Umkristallisation aus Wasser/Ethanol wird das 2-Cyclohexyl-6-methyl-anilin in reiner Form als Hydrochlorid erhalten. Fp: 54-55°C

b) Herstellung von 2-Chlor-3-cyclohexyl-toluol

Aus dem nach Absatz a) erhaltenen 2-Cyclohexyl-6-methyl-anilin erhält man analog Beispiel 1, Absatz a) in einer Ausbeute von 20 % 2-Chlor-3-cyclohexyl-toluol mit dem Kp = 73-75°C (0,1 mbar).

c) Elektrosynthese von 2-Chlor-3-cyclohexyl-benzylacetat

Man elektrolysiert das nach Absatz b) erhaltene 2-Chlor-3-cyclohexyl-toluol in der wie folgt beschriebenen Elektrolysezelle unter den angegebenen Bedingungen.

Apparatur: ungeteilte Zelle mit 11 bipolaren Elektroden

Anode: Graphit

Elektrolyt: 68 g (0,326 Mol) 2-Chlor-3-cyclohexyl-toluol, 350 g Acetanhydrid, 175 g $KSO_3C_6H_5$ und 2907 g Essigsäure

Kathode: Graphit

Stromdichte: 0,67 A/dm$^2$

Elektrolysetemperatur: 70°C

Elektrolyse mit 6 F/Mol 2-Chlor-3-cyclohexyl-toluol. Der Elektrolyt wird während der Elektrolyse mit 200 l/h durch die Zelle gepumpt.

Der Elektrolyseaustrag wird analog Beispiel 1, Absatz b) aufgearbeitet. Nach Reindestillation des Rückstandes bei 2 mbar und 145-165°C erhält man 0,9 g 2-Chlor-3-cyclohexyl-toluol und 29,4 g 2-Chlor-3-cyclohexyl-benzylacetat. Hieraus errechnet sich ein Umsatz von 98,7 % und eine Ausbeute von 33,7 %.

$^1$H-NMR (270 MHz, $CDCl_3$)

δ (ppm) = 1,2-1,5 (m, 5H, Ring-H); 1,7-1,9 (m, 5H, Ring-H); 2,1 (s, 3H, $CH_3COO-$); 3,06 (m, 1H Cyclohexyl-CH); 5,2 (s, 2H, $-CH_2OAc$); 7,2 (m, 3H, aromat.-H).

$^{13}$C-NMR (270 MHz, $CDCl_3$)

δ (ppm) = 20,8 (q); 26,3 (t); 26,9 (t, 2C); 33,2 (t, 2C); 40,6 (d); 64,4 (t); 126,7, 127,0 (d, 3C); 132,8 (s); 133,9 (s); 145,4 (s); 170,5 (s).

d) Herstellung von 2-Chlor-3-cyclohexyl-benzylalkohol

26,4g (99 mmol) 2-Chlor-3-cyclohexyl-benzylacetat tropft man zu einer Mischung aus 150 ml Ethanol und 8,4 g (150 mmol) Kaliumhydroxid. Nach 6 h bei 50°C engt man im Vakuum ein, versetzt den Rückstand mit

Wasser und extrahiert mit Dichlormethan. Nach üblicher Aufarbeitung der organischen Phase wird in Vakuum fraktioniert destilliert. Man erhält 17,4 g (78 %) 2-Chlor-3-cyclohexyl-benzylalkohol mit dem Kp = 133-135°C (0,5 mbar), $n_D^{20}$ = 1,5593.

$^1$H-NMR (300 MHz, $CDCl_3$)

δ (ppm) = 1,2-1,6 (m, 5H, Cyclohexyl-H); 1,7-2,0 (m, 5H, Cyclohexyl-H); 2,4 (s, 1H, OH); 3,1 (m, 1H, -CH<); 4,75 (s, 2H, $-CH_2OH$); 7,1-7,4 (m, 3H, aromat.-H).

Beispiel 6

3-tert.-Butyl-2-chlor-benzylalkohol

a) Elektrosynthese von 3-tert.-Butyl-2-chlor-benzylacetat

Man elektrolysiert 3-tert.-Butyl-2-chlor-toluol, das analog Beispiel 1, Absatz a) hergestellt wurde in der wie folgt beschriebenen Elektrolysezelle unter den angegebenen Bedingungen.

Apparatur: ungeteilte Zelle mit 11 bipolaren Elektroden

Anode: Graphit

Elektrolyt: 121 g (0,662 mol) 3-tert.-Butyl-2-chlor-toluol [$^1$H-NMR (300 MHz, $CDCl_3$); δ (ppm) = 1,5 (s, 9H); 2,2 (s, 3H); 7,0-7,4 (m, 3H)], 350 g Acetanhydrid, 175 g $KSO_3C_6H_5$ und 2854 g Essigsäure

Kathode: Graphit

Stromdichte: 1,33 A/dm$^2$

Elektrolysetemperatur: 70°C

Elektrolyse mit 4,5 F/Mol 3-tert.-Butyl-2-chlor-toluol.

Der Elektrolyt wird während der Elektrolyse mit 200 l/h durch die Zelle gempumpt.

Der Elektrolyseaustrag wird analog Beispiel 1, Absatz b) aufgearbeitet. Der Rückstand (205 g) enthält nach GC-Analyse 9,4 g 3-tert.-Butyl-2-chlor-toluol und 107 g 3-tert.-Butyl-2-chlor-benzylacetat. Hieraus errechnet sich ein Umsatz von 92,2 %, eine Ausbeute von 67,1 % und eine Selekvität von 72,8 %. 3-tert.-Butyl-2-chlor-benzylacetat wird bei 2 mbar und 112°C reindestilliert.

$^1$H-NMR (270 MHz, $CDCl_3$)

δ (ppm) = 1,5 (s, 9H, $-C(CH_3)_3$); 2,1 (s, 3H, $CH_3COO-$); 5,2 (s, 2H, $-CH_2OAc$); 7,15 (t, 1H, aromat.-H); 7,25 (d, 1H, aromat.-H); 7,38 (d, 1H, aromat.-H).

$^{13}$C-NMR (270 MHz, $CDCl_3$)

δ (ppm) = 20,6 (q); 29,9 (q, 3C); 36,4 (s); 64,6 (t); 126,4 (d); 127,2 (d); 127,5 (d); 133,1 (s); 135,7 (s); 147,1 (s); 170,1 (s).

b) Herstellung von 3-tert.-Butyl-2-chlor-benzylalkohol

Zu einer Lösung von 62,6 g (0,26 mol) 3-tert.-Butyl-2-chlor-benzylacetat in 400 ml Ethanol gibt man 21,0 g (0,39 mol) Kaliumhydroxid. Das Reaktionsgemisch wird 6 h bei 50°C gerührt, eingeengt und mit Wasser aufgenommen. Das wäßrige Gemisch wird mit Dichlormethan extrahiert. Aus der organischen Phase gewinnt man 51 g (quant.) 3-tert.-Butyl-2-chlor-benzylalkohol vom Kp. 103-105°C (0,2 mbar).

Beispiel 7

3-tert.-Butyl-2-fluor-benzylalkohol

a) Herstellung von 3-tert.-Butyl-2-fluor-toluol

In 500 ml 40 %ige Tetrafluoroborsäure tropft man bei 50°C 157 g (0,96 mol) 2-tert.-Butyl-6-methyl-anilin und anschließend eine Lösung von 66,2 g (0,96 mol) Natriumnitrit in 270 ml Wasser. Die Mischung wird 1 h bei 10°C gerührt. Das ausgefallene Diazoniumsalz wird abgesaugt, nacheinander mit 50 ml Eiswasser, 50 ml kaltem Ethanol und 50 ml Diethylether gewaschen und dann in 500 ml Paraffinöl suspendiert. Die Suspension wird in einen auf 150°C vorgeheizten Kolben getropft. Nach Beendigung der Gasentwicklung wird das Produkt durch Destillation über eine Claisendestille (Badtemperatur 140°C, Druck 26 mbar) bei einer Übergangstemperatur von 83°C gewonnen. Man erhält 47 g (29 %) 2-Fluor-3-tert.-butyl-toluol.

$^1$H-NMR ($CDCl_3$)

δ (ppm) = 1,38 (s, 9H, $C(CH_3)_3$; 2,22 (s, 3H, $Ar-CH_3$); 6,82-6,98 (m, 2H, aromat. H); 7,05-7,12 (m, 1H, aromat. H).

b) Elektrosynthese von 3-tert.-Butyl-2-fluor-benzylacetat

Man elektrolysiert das nach Absatz a) erhaltene 3-tert.-Butyl-2-fluor-toluol in der wie folgt beschriebenen Elektrolysezelle unter den angegebenen Bedingungen.

Apparatur: ungeteilte Zelle mit 11 bipolaren Elektroden

Anode: Graphit

Elektrolyt: 50 g (0,301 Mol) 3-tert.-Butyl-2-fluor-toluol, 300 g Acetanhydrid, 150 g $KSO_3C_6H_5$ und 2600 g Essigsäure

Kathode: Graphit

Stromdichte: 0,67 A/$dm^2$

Elektrolysetemperatur: 70°C

Elektrolyse mit 5 F/Mol 3-tert.-Butyl-2-fluor-toluol.

Der Elektrolyt wird während der Elektrolyse mit 200 l/h durch die Zelle gepumpt.

Der Elektrolyseaustrag wird analog Beispiel 1, Absatz b) aufgearbeitet. Der Rückstand (110 g) enthält nach GC-Analyse 1,3 g 3-tert.-Butyl-2-fluor-toluol und 45,9 g 3-tert.-Butyl-2-fluor-benzylacetat. Hieraus errechnet sich ein Umsatz von 97,4 %, eine Ausbeute von 68,3 % und eine Selektivität von 69,9 %.

3-tert.-Butyl-2-fluor-benzylacetat wird bei 4 mbar und 105°C reindestilliert.

$^1H$-NMR (270 MHz, $CDCl_3$)

δ (ppm) = 1,38 (s, 9H, $-C(C\underline{H}_3)_3$); 2,05 (s, 3H, $CH_3COO-$); 5,18 (s, 2H, $-CH_2OAc$); 7,0 (t, 1H, aromat.-H); 7,22 (m, 2H, aromat.-H).

$^{13}C$-NMR (270 MHz, $CDCl_3$)

δ (ppm) = 20,7 (q); 30,1 (q, 3C); 60,4 (t); 123,6 (d); 124,2 (s); 127,5 (d); 128,2 (d); 137,4 (s); 160,3 (s); 170,4 (s).

c) Herstellung von 3-tert.-Butyl-2-fluor-benzylalkohol

Man gibt zu einer Lösung von 25 g (0,11 mol) 3-tert.-Butyl-2-fluor-benzylacetat in 100 ml Dioxan 67 g 8 %ige wäßrige Natronlauge und rührt das Gemisch 7 h bei 50°C. Die Mischung wird mit 100 ml Wasser verdünnt und dreimal mit Dichlormethan extrahiert. Nach dem Trocknen und Einengen verbleiben 19,5 g (97 %) 3-tert.-Butyl-2-fluor-benzylalkohol.

$^1H$-NMR ($CDCl_3$)

δ (ppm) = 1,38 (s, 9H, $C(CH_3)_3$); 2,21 (s, 1H, -OH); 4,71 (s, 2H, $-CH_2-$); 7,00-7,09 (m, 1H, aromat. H); 7,18-7,30 (m, 2H, aromat. H).

Beispiel 8

2-Chlor-3-methyl-benzylalkohol

a) Elektrosynthese von 2-Chlor-3-methyl-benzylacetat

Man elektrolysiert 2-Chlor-1,3-dimethyl-benzol in der wie folgt beschriebenen Elektrolysezelle unter den angegebenen Bedingungen.

Apparatur: ungeteilte Zelle mit 11 bipolaren Elektroden

Anode: Graphit

Elektrolyt: 115 g (0,819 Mol) 2-Chlor-1,3-dimethyl-benzol, 350 g Acetanhydrid, 175 g $KSO_3C_6H_5$ und 2860 g Essigsäure

Kathode: Graphit

Stromdichte: 0,67 A/$dm^2$

Elektrolysetemperatur: 70°C

Elektrolyse mit 4,5 F/Mol 2-Chlor-1,3-dimethyl-benzol.

Der Elektrolyt wird während der Elektrolyse mit 200 l/h durch die Zelle gepumpt.

Der Elektrolyseaustrag wird analog Beispiel 1, Absatz b) aufgearbeitet. Nach Rückstand (200 g) enthält nach GC-Analyse 0,8 g 2-Chlor-1,3-dimethyl-benzol und 77 g 2-Chlor-3-methyl-benzylacetat. Hieraus errechnet sich ein Umsatz von 99,3 %, eine Ausbeute von 47,4 % und eine Selektivität von 47,7 %. 2-Chlor-3-methyl-benzylacetat wird bei 2 mbar und 87-90°C reindestilliert.

$^1H$-NMR (270 MHz, $CDCl_3$)

δ (ppm) = 2,08 (s, 3H, $CH_3COO-$); 2,35 (s, 3H, $CH_3$-Ar); 5,2 (s, 2H, $-CH_2OAc$); 7,05-7,22 (m, 3H, aromat.-H).

$^{13}C$-NMR (270 MHz, $CDCl_3$)

δ (ppm) = 20,3 (q); 20,8 (q); 64,0 (t); 126,3 (d); 127,1 (d); 130,6 (d); 133,6 (s); 133,8 (s); 136,6 (s); 170,4 (s).

b) Herstellung von 2-Chlor-3-methyl-benzylalkohol

68 g (0,34 mol) des nach Absatz a) erhaltenen Benzylacetats werden in einer Mischung aus 640 ml Dioxan und 65 ml 10 %iger Natronlauge analog Beispiel 1, Absatz c) verseift und aufgearbeitet. Man erhält 43 g (81 %) 2-Chlor-3-methyl-benzylalkohol vom Fp. 63-64°C.

Beispiel 9

Elektrosynthese von 2-Chlor-3-isopropyl-benzylpropionat

Man elektrolysiert 2-Chlor-3-isopropyl-toluol in der wie folgt beschriebenen Elektrolysezelle unter den angegebenen Bedingungen.

Apparatur: ungeteilte Zelle mit 11 bipolaren Elektroden
Anode: Graphit
Elektrolyt: 242 g (1,436 Mol) 2-Chlor-3-isopropyl-toluol, 125 g $(Et_3NMe)SO_4Me$ und 2133 g Propionsäure
Kathode: Graphit
Stromdichte: 0,67 $A/dm^2$
Elektrolysetemperatur: 70°C
Elektrolyse mit 8 F/Mol 2-Chlor-3-isopropyl-toluol.
Der Elektrolyt wird während der Elektrolyse mit 200 l/h durch die Zelle gepumpt.

Der Elektrolyseaustrag wird analog Beispiel 1, Absatz b) aufgearbeitet. Der Rückstand (200 g) enthält nach GC-Analyse 9,2 g 2-Chlor-3-isopropyl-toluol und 156,2 g 2-Chlor-3-isopropyl-benzylpropionat. Hieraus errechnet sich ein Umsatz von 96,2 %, eine Ausbeute von 47,0 % und eine Selektivität von 48,9 %. 2-Chlor-3-isopropyl-benzylpropionat wird bei 2 mbar und 117°C reindestilliert.

$^1H$-NMR (270 MHz, $CDCl_3$)

δ (ppm) = 1,16 (t, 3H, $C\underline{H}_3CH_2COO$-); 1,22 (d, 6H, -$CH(C\underline{H}_3)_2$); 2,37 (q, 2H, $CH_3C\underline{H}_2COO$-); 3,45 (Septett, 1H, -$C\underline{H}(CH_3)_2$); 5,2 (s, 2H, $ArCH_2O$-); 7,2 (m, 3H, aromat.-H).

$^{13}C$-NMR (270 MHz, $CDCl_3$)

δ (ppm) = 9,1 (q); 22,7 (q, 2C); 27,6 (t); 30,3 (d); 64,1 (t); 126,4 (d); 126,8 (d); 127,0 (d); 132,8 (s); 134,4 (s); 146,5 (s); 173,7 (s).

Beispiel 10

Elektrosynthese von 2-Chlor-3-isopropyl-benzylformiat

Man elektrolysiert 2-Chlor-3-isopropyl-toluol in der wie folgt beschriebenen Elektrozelle unter den angegebenen Bedingungen.

Apparatur: ungeteilte Zelle mit 11 bipolaren Elektroden
Anode: Graphit
Elektrolyt: 250 g (1,484 Mol) 2-Chlor-3-isopropyl-toluol, 1400 g Acetonitril, 125 g $(Et_3NMe)SO_4Me$ und 2125 g Ameisensäure
Kathode: Graphit
Stromdichte: 1,33 $A/dm^2$
Elektrolysetemperatur: 70°C
Elektrolyse mit 16 F/Mol 2-Chlor-3-isopropyl-toluol.
Der Elektrolyt wird während der Elektrolyse mit 200 l/h durch die Zelle gepumpt.

Der Elektrolyseaustrag wird analog Beispiel 1, Absatz b) aufgearbeitet. Der Rückstand (291 g) enthält nach GC-Analyse 128,3 g 2-Chlor-3-isopropyl-toluol und 96,0 g 2-Chlor-3-isopropyl-benzylformiat. Hieraus errechnet sich ein Umsatz von 48,7 %, eine Ausbeute von 30,4 % und eine Selektivität von 62,4 %. 2-Chlor-3-isopropyl-benzylformiat wird bei 2 mbar und 103-107°C reindestilliert.

$^1H$-NMR (270 MHz, $CDCl_3$)

δ (ppm) = 1,2 (d, 6H, -$CH(C\underline{H}_3)_2$); 3,45 (Septett, 1H, -$C\underline{H}(CH_3)_2$); 5,27 (s, 2H, -$CH_2OCHO$); 7,2 (m, 3H, aromat.-H); 8,06 (s, 1H, -OCHO).

$^{13}C$-NMR (270 MHz, $CDCl_3$)

δ (ppm) = 22,6 (q, 2C); 30,3 (d); 63,7 (t); 126,7 (d); 126,9 (d); 127,4 (d); 132,9 (s); 133,5 (s); 146,6 (s); 160,5 (d).

## Patentansprüche

1. Verfahren zur Herstellung von Benzolderivaten der allgemeinen Formel

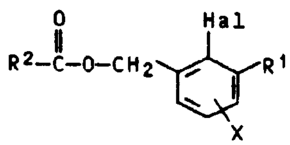

I,

in der Hal ein Halogenatom, $R^1$ einen geradkettigen, verzweigten oder ringförmigen Kohlenwasserstoffrest mit 1 bis 18 C-Atomen, $R^3$ ein Wasserstoffatom oder einen Alkylrest mit 1 bis 6 C-Atomen und X ein Wasserstoff- oder Halogenatom bedeuten, dadurch gekennzeichnet, daß man Toluolderivate der allgemeinen Formel

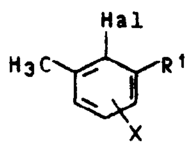

II,

in der Hal, $R^1$ und X die obengenannte Bedeutung haben, in Gegenwart einer Säure der Formel $R^2$-COOH (III) elektrochemisch oxidiert.

2. Benzolderivate der allgemeinen Formel

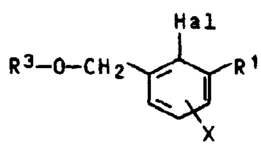

IV,

in der Hal ein Halogenatom, $R^1$ einen geradkettigen, verzweigten oder ringförmigen Kohlenwasserstoffrest mit 1 bis 18 C-Atomen, $R^3$ ein Wasserstoffatom oder einen $R^2$-CO-Rest, $R^2$ ein Wasserstoffatom oder einen Alkylrest mit 1 bis 6 C-Atomen und X ein Wasserstoff- oder Halogenatom bedeuten, wobei jedoch $R^1$ nicht Methyl sein kann, wenn X Wasserstoff bedeutet.

3. Benzolderivate der allgemeinen Formel

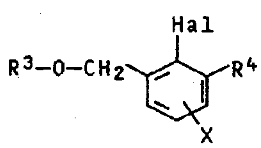

V,

in der Hal ein Fluor-, Chlor- oder Bromatom und $R^4$ einen verzweigten oder ringförmigen Alkylrest mit 3 bis 12 C-Atomen bedeuten und $R^3$ und X die in Anspruch 2 genannte Bedeutung haben.

4. Verwendung der im Anspruch 2 gekennzeichneten Benzolderivate der Formel IV, in der $R^3$ für den $R^2$-CO-Rest steht, für die Herstellung von Benzylalkoholen der allgemeinen Formel

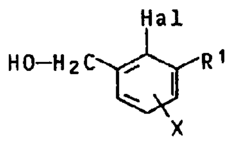

VII,

in der Hal, $R^1$ und X die in Anspruch 1 genannte Bedeutung haben.

## Claims

1. A process for the preparation of a benzene derivative of the formula

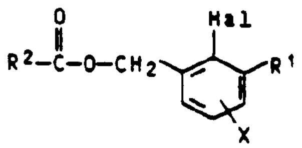

I

where Hal is halogen, $R^1$ is a straight-chain, branched or cyclic hydrocarbon radical of 1 to 18 carbon atoms, $R^2$ is hydrogen or alkyl of 1 to 6 carbon atoms and X is hydrogen or halogen, wherein a toluene derivative of the formula

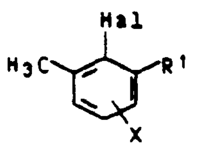

II

where Hal, $R^1$ and X have the abovementioned meanings, is electrochemically oxidized in the presence of an acid of the formula $R^2$-COOH (III).

2. A benzene derivative of the formula

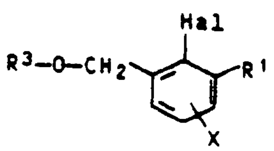

IV

where Hal is halogen, $R^1$ is a straight-chain, branched or cyclic hydrocarbon radical of 1 to 18 carbon atoms, $R^3$ is hydrogen or an $R^2$-CO- radical, $R^2$ is hydrogen or alkyl of 1 to 6 carbon atoms and X is hydrogen or halogen, but $R^1$ cannot be methyl if X is hydrogen.

3. A benzene derivative of the formula

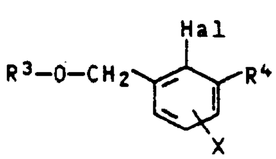

V

where Hal is fluorine, chlorine or bromine, $R^4$ is a branched or cyclic alkyl radical of 3 to 12 carbon atoms, and $R^3$ and X have the meanings stated in claim 2.

4. Use of a benzene derivative of the formula IV as defined in claim 2, where $R^3$ is an $R^2$ -CO- radical, for the preparation of a benzyl alcohol of the formula

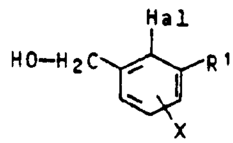 VII,

where Hal, $R^1$ and X have the meanings stated in claim 1.

## Revendications

1.- Procédé de préparation de dérivés du benzène de formule générale

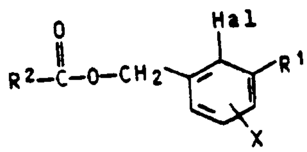 I,

dans laquelle Hal représente un atome d'halogène, $R^1$ représente un reste hydrocarboné à chaîne droite, ramifié ou cyclique à 1-18 atomes de carbone, $R^2$ représente un atome d'hydrogène ou un reste alkyle à 1-6 atomes de carbone et X représente un atome d'hydrogène ou d'halogène, caractérisé en ce qu'on soumet à une oxydation électrochimique des dérivés du toluène de formule générale

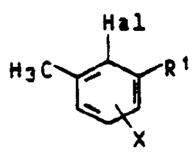 II,

dans laquelle Hal, $R^1$ et X ont les significations sus-indiquées, en présence d'un acide de formule $R^2$-COOH (III).

2.- Dérivés du benzène de formule générale

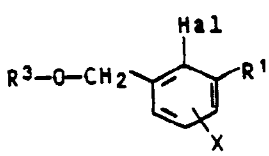 IV,

dans laquelle Hal représente un atome d'halogène, $R^1$ représente un reste hydrocarboné à chaîne droite, ramifié ou cyclique à 1-18 atomes de carbone, $R^3$ représente un atome d'hydrogène ou un reste $R^2$-CO, $R^2$ représentant un atome d'hydrogène ou un reste alkyle à 1-6 atomes de carbone et X représente un atome d'hydrogène ou d'halogène, $R^1$ ne pouvant toutefois pas être un reste méthyle lorsque X est un atome d'hydrogène.

3.- Derivés du benzène de formule genérale

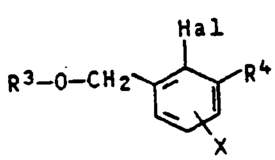 V,

dans laquelle Hal représente un atome de fluor, de chlore ou de brome, $R^4$ représente un reste alkyle ramifié ou cyclique à 3-12 atomes de carbone et $R^3$ et X ont les significations indiquées dans la revendication 2.

4.- Utilisation des dérivés du benzène de formule IV caractérisés dans la revendication 2, dans lesquels $R^3$ est mis pour le reste $R^2$-CO, pour la préparation d'alcools benzyliques de formule générale

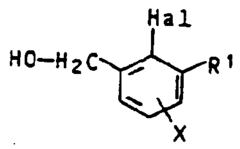

VII,

dans laquelle Hal, $R^1$ et X ont les significations indiquées dans la revendication 1.